# EUROPEAN PATENT SPECIFICATION

(11) **EP 3 035 944 B1**
(45) Date of publication and mention of the grant of the patent: **02.10.2019**
(21) Application number: 14781306.7
(22) Date of filing: 22.08.2014
(51) Int. Cl.: A61K 35/74, A61P 43/00, A61K 35/741

(54) **PROBIOTICS FOR USE IN THE TREATMENT OF BACTERIAL VAGINOSIS**
PROBIOTIKA ZUR VERWENDUNG BEI DER BEHANDLUNG BAKTERIELLER VAGINOSE
PROBIOTIQUES POUR SON UTILISATION DANS LE TRAITEMENT DE LA VAGINOSE BACTÉRIENNE

(30) Priority: 22.08.2013 EP 13181363; 07.04.2014 EP 14163695
(43) Date of publication of application: 29.06.2016
(73) Proprietor: Nederlandse Organisatie voor toegepast- natuurwetenschappelijk onderzoek TNO, 2595 DA 's-Gravenhage (NL)
(72) Inventor: KORT, Remco, NL-2595 DA `s-Gravenhage (NL); SCHUREN, Frank Henri Johan, NL-2595 DA `s-Gravenhage (NL)
(74) Representative: V.O.
(86) International application number: PCT/NL2014/050570
(87) International publication number: WO 2015/026235

(56) References cited:
- US-A1- 2003 118 571
- FALAGAS M E ET AL: "Probiotics for the treatment of women with bacterial vaginosis", CLINICAL MICROBIOLOGY AND INFECTION, WILEY-BLACKWELL PUBLISHING LTD, UNITED KINGDOM, SWITZERLAND, vol. 13, no. 7, 1 July 2007 (2007-07-01), pages 657-664, XP002631583, ISSN: 1198-743X, DOI: 10.1111/J.1469-0691.2007.01688.X [retrieved on 2007-03-02]
- TEITELBAUM JONATHAN E ET AL: "Nutritional impact of pre- and probiotics as protective gastrointestinal organisms", ANNUAL REVIEW OF NUTRITION, ANNUAL REVIEWS INC., PALO ALTO, CA, US, vol. 22, 1 January 2002 (2002-01-01), pages 107-138, XP009107660, ISSN: 0199-9885, DOI: 10.1146/ANNUREV.NUTR.22.110901.145412
- Whiteman, H. (2013, August 8).: ""Fewer 'good gut' bacteria in C-section infants", Medical News Today , 8 August 2013 (2013-08-08), pages 1-2, XP002735472, Retrieved from the Internet: URL:http://www.medicalnewstoday.com/articl es/264473.php [retrieved on 2014-12-02]
- J. RAVEL ET AL: "Vaginal microbiome of reproductive-age women", PROCEEDINGS OF THE NATIONAL ACADEMY OF SCIENCES, vol. 108, no. Supplement_1, 15 March 2011 (2011-03-15), pages 4680-4687, XP055083668, ISSN: 0027-8424, DOI: 10.1073/pnas.1002611107
- A. EVERARD ET AL: "Cross-talk between Akkermansia muciniphila and intestinal epithelium controls diet-induced obesity", PROCEEDINGS OF THE NATIONAL ACADEMY OF SCIENCES, vol. 110, no. 22, 28 May 2013 (2013-05-28) , pages 9066-9071, XP055074467, ISSN: 0027-8424, DOI: 10.1073/pnas.1219451110
- P. B. ECKBURG: "Diversity of the Human Intestinal Microbial Flora", SCIENCE, vol. 308, no. 5728, 10 June 2005 (2005-06-10), pages 1635-1638, XP055050950, ISSN: 0036-8075, DOI: 10.1126/science.1110591
- STRATIKI ET AL: "The effect of a bifidobacter supplemented bovine milk on intestinal permeability of preterm infants", EARLY HUMAN DEVELOPMENT, SHANNON, IR, vol. 83, no. 9, 14 September 2007 (2007-09-14), pages 575-579, XP022245155, ISSN: 0378-3782, DOI: 10.1016/J.EARLHUMDEV.2006.12.002
- SAAVEDRA J M: "Use of probiotics in pediatrics: Rationale, mechanisms of action, and practical aspects", NUTRITION IN CLINICAL PRACTICE, WILLIAMS AND WLIKINS, BALTIMORE, MD, US, vol. 22, no. 3, 1 June 2007 (2007-06-01), pages 351-365, XP009129949, ISSN: 0884-5336, DOI: 10.1177/0115426507022003351
- HEDVIG E JAKOBSSON ET AL: "Decreased gut microbiota diversity, delayed Bacteroidetes colonisation and reduced Th1 responses in infants delivered by Caesarean section", GUT, vol. 63, no. 4, 7 August 2013 (2013-08-07) , pages 559-566, XP009181476, ISSN: 0017-5749 [retrieved on 2013-08-07]

## Description

### FIELD OF THE INVENTION

The invention relates to the fields of food, medicine, bacteriology and health science. More particular, the invention relates to the field of bacterial-based healthy foods and/or treatment by administration of bacterial cultures.

### BACKGROUND OF THE INVENTION

Probiotics, are defined by the FAO and the WHO as living microorganisms which, when administered in adequate amounts, confer a beneficial health effect on the host. Although in most cases administration is given orally, the definition does not exclude other modes of administration. Addition of probiotic bacteria to the diet is an approach for increasing the health of the individual human or animal by promoting the presence of beneficial bacteria in the intestine.

The roles, which probiotics have on microbe-microbe interactions include among others competition for nutrients and colonization areas with harmful micro-organisms including pathogenic bacteria, such as *Clostridium difficile, C. perfringens, E. coli,* etc.. Probiotics can act for example by production of antimicrobial substances, such as bacteriocins and/or the generation of restrictive physiological conditions, including acidification, caused by lactic acid and other fermentations (Teitelbaum et al. 2002. Nutritional impact of pre- and probiotics as protective gastrointestinal organisms. Annual Reviews Nutrition 22:107-138).

Use of probiotics as dietary intervention is partly based on the concept that specific strains selected from the healthy gut microbiota may have powerful anti-pathogenic and anti-inflammatory properties, and therefore may provide resistance to intestinal diseases (Isolauri et al. 2002. Probiotics: a role in the treatment of intestinal infection and inflammation? Gut 50:54-59). In monogastric animals, including humans, commensal microbiota contribute to intestinal protection against pathogens by competition for nutrients and pathogen binding sites, and/or regulation of immune response.

Many probiotic compositions are known, since early history. The oldest probiotics are components of products we now recognize as 'normal' fermented foods, such as yoghurt, tempeh and some juices and soy beverages. An overview of medically used probiotics has been given by Blandino, G. et al., 2008, Probiotics: overview of microbiological and immunological characteristics, Expert Rev. Anti Infect. Ther. 6:497-508.

Normally probiotics are provided by culturing a specific micro-organism or, at best, a mixture of specific micro-organisms. If these are administered to combat conditions of dysbiosis, these probiotics are able to 'normalise' the microbiota. Recently, it has been found that it is also feasible - and probably more preferable, to provide one (or more) of the patient's own species of micro-organisms. Such a scheme would fit into today's development of personalised therapeutics'. One example has been described in US 2003/118,571 in which a micro-organism is isolated from a human sample and cultured and, after sufficient culturing, supplied to that same human. In that same document it has also been proposed that microbiota restoration ,especially urogenital microbiota restoration can be achieved with a bacterial species that has been isolated from that patient. For this, when the patient in need of microbiota restoration or maintenance is healthy, urogenital organisms are recovered, cultured and the main healthy species isolated and stored. If and when the person has a depleted urogenital microbiota at some later point in his/her life, such as during pregnancy or during a urogenital infection, the originally isolated organisms are cultured, and re-implanted vaginally or re-administered orally. Such an approach would both personalize the therapy and utilize organisms which are known to have been associated with the person's health and recognized as "self"-organisms by their immune system at one stage in life.

However, one disadvantage of this method is that the micro-organisms need to be sampled, isolated and stored while the person is (still) healthy.

Therefore, there is still need for alternative methods for providing personalised probiotic therapy.

### SUMMARY OF THE INVENTION

Herein described is a method for treatment of a dysbiotic condition in a subject comprising the steps of:
a. obtaining a sample from said subject wherein said sample is obtained from the location of the dysbiotic condition and wherein said sample comprises micro-organisms;
b. *in vitro* culturing of said sample under conditions that favour beneficial micro-organisms;
c. re-introducing a sample of said cultured micro-organisms into said subject.

Alternatively, herein described is a probiotic composition for use in the treatment of a dysbiotic condition in a subject, wherein said composition has been obtained by culturing a sample comprising micro-organisms from the location of the dysbiotic condition from said subject under conditions that favour beneficial micro-organisms. Preferably such a probiotic composition comprises additional excipients. Further preferable, this probiotic composition is suitable for administration to the location of the dysbiotic condition.

In a preferred embodiment of the present invention, the subject is a human. In another preferred embodiment said subject is an animal, preferably chosen from the group of cattle, horses, poultry, pigs, dogs and cats.

The location of the probiotic condition may be chosen from the intestine, the vagina, the oral cavity, the respiratory tract and the skin.

The micro-organisms in the sample that is taken from the subject and cultured are bacteria. Further preferred, the beneficial micro-organisms are selected from the group of lactic acid bacteria and the genera *Akkermansia* and *Bacteroides.*

Also preferred is the embodiment wherein the *in vitro* culture is maintained for more than two days.

In a further preferred embodiment the re-introduction of the cultured bacteria is performed by oral or topical administration.

Said cultured, enriched sample as obtained in step b)is stored and a sample of said stored sampled is used for administration to said subject in case of a recurrent dysbiotic condition, such as a recurrent infection.

Herein described is a method to prevent the occurrence of an auto-immune disease in children delivered through Caesarean section, comprising:
a. taking a vaginal microbiota sample from the mother at the time of delivery, or shortly before or after this time;
b. optionally culturing said sample, during which culture said sample may optionally be enriched for beneficial micro-organisms;
c. administration of at least a part of said sample obtained from step a or step b to the new-born child, preferably by oral administration.
The invention is defined in claims 1-6.

### BRIEF DESCRIPTION OF THE DRAWINGS

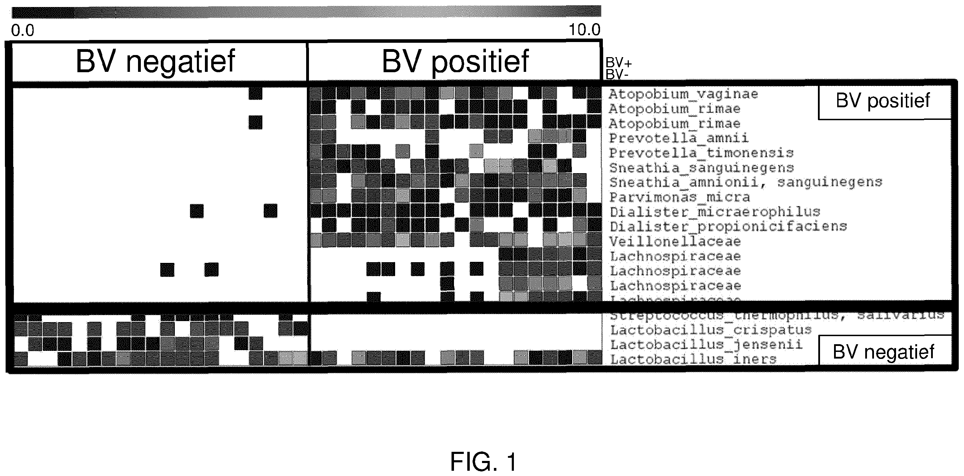

Figure 1. Identification of bacterial vaginosis negative (BV-) (Nugent scoring 0-3) and BV+ (Nugent scoring 8 - 10) associated species by the cultivation-independent 454 pyrosequencing of 16S rRNA. Rows represent the abundant genera and species in a subject, for this study a total of 20 BV- and 20 BV+ subjects were selected.

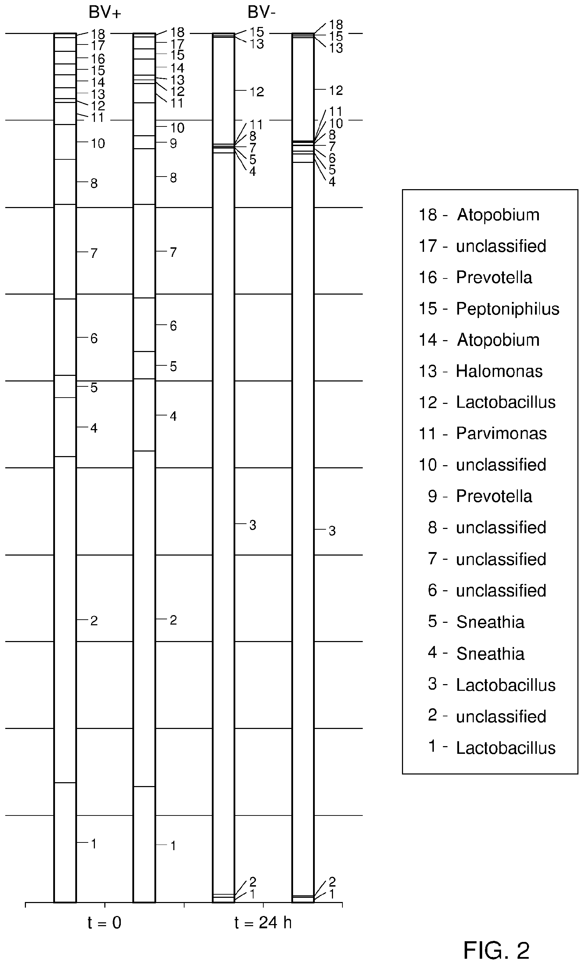

Figure 2. Typical genera associated with bacterial vaginosis have been replaced by a microflora known to be predominantly present in healthy women. Data shown from two individuals, with at the left side an overview of bacterial species in the sample at t=0, and at the right side the bacterial species that were found after culturing for 24 hours in modified CDM medium.

### DETAILED DESCRIPTION OF THE INVENTION

### Definitions

The term "probiotic composition" as used herein refers to a composition comprising one or more probiotic organisms and one or more acceptable excipients suitable for application to a mammal. It will be appreciated that acceptable excipients will be well known to the person skilled in the art of probiotic composition preparation. Examples of such acceptable excipients for oral administration of the probiotic composition include: sugars such as sucrose, isomerized sugar, glucose, fructose, palatinose, trehalose, lactose and xylose; sugar alcohols such as sorbitol, xylitol, erythritol, lactitol, palatinol, reduced glutinous starch syrup and reduced glutinous maltose syrup; polysaccharides as maltodextrins, starches like maize starch, rice starch, potato starch and wheat starch, emulsifiers such as sucrose esters of fatty acid, glycerin esters of fatty acid and lecithin; thickeners (stabilizers) such as carrageenan, xanthan gum, guar gum, pectin and locust bean gum; acidifiers such as citric acid, lactic acid and malic acid; fruit juices such as lemon juice, orange juice and berry juice; vitamins such as vitamin A, vitamin B, vitamin C, vitamin D and vitamin E; and minerals such as calcium, iron, manganese and zinc. For topical administration of a probiotic composition the composition can additionally or alternatively comprise polymers, such as natural polymers including proteins such as zein, modified zein, casein, gelatin, gluten, serum albumin, and collagen, polysaccharides such as cellulose, dextrans, and polyhyaluronic acid, or synthetic polymers including polyphosphazenes, poly(vinyl alcohols), polyamides, polycarbonates, polyacrylates, polyalkylenes, polyacrylamides, polyalkylene glycols, polyalkylene oxides, polyalkylene terephthalates, polyvinyl ethers, polyvinyl esters, polyvinyl halides, polyvinylpyrrolidone, polyglycolides, polysiloxanes, polyurethanes and copolymers thereof. Examples of suitable polyacrylates include poly(methyl methacrylate), poly(ethyl methacrylate), poly(butyl methacrylate), poly(isobutyl methacrylate), poly(hexyl methacrylate), poly(isodecyl methacrylate), poly(lauryl methacrylate), poly(phenyl methacrylate), poly(methyl acrylate), poly(isopropyl acrylate), poly(isobutyl acrylate) and poly(octadecyl acrylate). Synthetically modified natural polymers include cellulose derivatives such as alkyl celluloses, hydroxyalkyl celluloses, cellulose ethers, cellulose esters, and nitrocelluloses. Examples of suitable cellulose derivatives include methyl cellulose, ethyl cellulose, hydroxypropyl cellulose, hydroxypropyl methyl cellulose, hydroxybutyl methyl cellulose, cellulose acetate, cellulose propionate, cellulose acetate butyrate, cellulose acetate phthalate, carboxymethyl cellulose, cellulose triacetate and cellulose sulfate sodium salt.Also usable are degradable polymers, such as polysaccharides such as alginate, dextran, cellulose, collagen, and chemical derivatives thereof (substitutions, additions of chemical groups, for example, alkyl, alkylene, hydroxylations, oxidations, and other modifications routinely made by those skilled in the art), and proteins such as albumin, zein and copolymers and blends thereof, alone or in combination with synthetic polymers. It is also possible that the probiotic is administered in the form of a hydrogel. Suitable hydrogels can be formed from synthetic polymers such as polyethylene glycol, polyethylene oxide, polyvinyl alcohol, polyvinyl pyrrolidone, polyacrylates, poly (ethylene terephthalate), poly(vinyl acetate), and copolymers and blends thereof, as well as natural polymers such as cellulose and alginate, as described above.

Probiotic compositions for oral use may be advantageously provided in the form of a dairy product, such as yoghurt or curd.

Probiotic compositions for topical administration may be applied in the form of crèmes or ointments. As such they will ideally comprise an oily substance originating from vegetable, marine or animal sources. Suitable liquid oil includes saturated, unsaturated or polyunsaturated oils. By way of example, the unsaturated oil may be olive oil, corn oil, soybean oil, canola oil, cottonseed oil, coconut oil, sesame oil, sunflower oil, borage seed oil, syzigium aromaticum oil, hempseed oil, herring oil, cod-liver oil, salmon oil, flaxseed oil, wheat germ oil, evening primrose oils or mixtures thereof, in any proportion. These crèmes or ointments may further comprise poly-unsaturated fatty acids. In one or more embodiments, said unsaturated fatty acids are selected from the group of omega-3 and omega-6 fatty acids. Examples of such polyunsaturated fatty acids are linoleic and linolenic acid, gamma-linoleic acid (GLA), eicosapentaenoic acid (EPA) and docosahexaenoic acid (DHA). Such unsaturated fatty acids are known for their skin-conditioning effect, which contribute to the therapeutic benefit of the composition. Thus, the composition can include at least 6% of an oil selected from omega-3 oil, omega-6 oil, and mixtures thereof. Also usable are the essential oils, which are also considered therapeutically active oils, which contain active biologically occurring molecules and, upon topical application, exert a therapeutic effect, which is conceivably synergistic to the beneficial effect of the probiotic mixture in the composition. Another class of therapeutically active oils includes liquid hydrophobic plant-derived oils, which are known to possess therapeutic benefits when applied topically. Silicone oils also may be used and are desirable due to their known skin protective and occlusive properties. Suitable silicone oils include non-volatile silicones, such as polyalkyl siloxanes, polyaryl siloxanes, polyalkylaryl siloxanes and polyether siloxane copolymers, polydimethylsiloxanes (dimethicones) and poly(dimethylsiloxane)-(diphenyl-siloxane) copolymers. These are chosen from cyclic or linear polydimethylsiloxanes containing from about 3 to about 9, preferably from about 4 to about 5, silicon atoms. Volatile silicones such as cyclomethicones can also be used. Silicone oils are also considered therapeutically active oils, due to their barrier retaining and protective properties.

The term "dysbiosis" is defined as a state in which the microbiota produces harmful effects via (a) qualitative and quantitative changes in the content or amount of the microbiota itself, (b) changes in their metabolic activities; and/or (c) changes in their local distribution. Specifically, dysbiosis of the vagina is defined as an aberration of the healthy state. A healthy state is defined in this case as a condition with a relatively low susceptibility to sexually transmitted diseases. It has been widely accepted that the activity of *Lactobacillus* spp. contributes to maintain this state through the protection of the vaginal environment against pathogens by the production of lactic acid, resulting in a low pH. Hence, vaginal dysbiosis is, amongst others, characterized by the presence of a relative high pH.

The term "microbiota" as used herein denominates the community of commensal microorganisms that colonise (parts of) an organ of the host, such as the vagina, the oral cavity, the skin, the lungs or the gastro-intestinal tract, together with the food-ingested, or transient microorganisms. However, these transient microbiota are not regarded as part of the so-called 'indigenous microbiota'.
"Epithelium" or "epithelial cell or tissue" as used herein is one of the four basic types of animal tissue, along with connective tissue, muscle tissue and nervous tissue. Epithelial tissues line the cavities and surfaces of structures throughout the body, and also form many glands. Functions of epithelial cells include secretion, selective absorption, protection, transcellular transport and detection of sensation.

"Mucosa" as used herein is the term used to indicate a mucous membrane. Mucus (adjectival form: "mucous") is a slippery secretion produced by, and covering, mucous membranes. Mucous fluid is typically produced from cells found in mucous glands. Mucous cells secrete products that are rich in glycoproteins and water. Mucous fluid may also originate from mixed glands, which contain both serous and mucous cells. It is a viscous colloid containing antiseptic enzymes (such as lysozyme), immunoglobulins, inorganic salts, proteins such as lactoferrin, and glycoproteins known as mucins that are produced by goblet cells in the mucous membranes and submucosal glands. This mucus serves to protect epithelial cells (the lining of the tubes) in the respiratory, gastrointestinal, urogenital, visual, and auditory systems in mammals;

In a healthy animal, the internal tissues, e.g. blood, brain, muscle, etc., are normally free of microorganisms. However, the surface tissues, i.e., skin and mucous membranes, are constantly in contact with environmental organisms and become readily colonized by various microbial species. The mixture of organisms regularly found at any anatomical site is referred to as the normal flora, or preferably "indigenous microbiota". The normal flora of humans consists of a few eukaryotic fungi and protists, but bacteria are the most numerous and obvious microbial components of the normal flora. Although humans are exposed to a wide variety of microbes throughout their lives, only a limited number of species are able to permanently colonize the various body sites available, and each body site harbors a microbial community comprised of certain characteristic species. Residents of a body site should be able to grow and reproduce under the conditions operating at the site, whereas organisms that cannot do so, are regarded as transients. In most cases the residents adhere to a substrate within the body site - this may be (1) a host epithelial cell, (2) components of the extracellular matrix (mucus), (3) another microbe or (4) a structure produced by the host (e.g. a tooth).

### Detailed description of the embodiments of the invention

In case a patient suffers a condition of dysbiosis the therapy should be pointed at the recovery of the local microbiota balance. Known therapies centre around administration of cultured microbiological species, where a single micro-organism is prepared to restore the balance. In some cases probiotic mixtures are available, but mostly monocultures have been applied.
In recent years, especially for dysbiosis of the intestinal microbiota a therapy in which a complete sample of micro-organisms was taken from a healthy subject and transplanted into the gut of a person in need thereof has become increasingly popular. However, it appears that there is quit a large variation from subject to subject in the composition of the microbiota. The individualisation of microbiota has been evidenced in several recent studies (e.g. Faith, J.J. et al.., Science 341: 1237439).In these studies it appeared that strains, defined as isolates sharing >96% of their genome content, were maintained over time within an individual and between family members but not between unrelated individuals. Thus, early gut colonizers, such as those acquired from our parents and siblings, have the potential to exert their physiologic, metabolic, and immunologic effects for most, and perhaps all, of our lives.
The immune system and the relation of the mucosal tissues therewith seems to be pivotal in the regulation of this system. This has been demonstrated by Sharma et al. (J. Biomed. Biotechnol., 2010, 305879) who studied the function of the gut-barrier to colonisation by micro-organisms. They concluded that the complex integration and execution of this function are superbly carried out by the intestinal mucosal (IM) surface. Exposed to trillions of luminal microbes, the IM averts threats by signalling to the innate immune system, through pattern recognition receptors (PRR), to respond to the commensal bacteria by developing tolerance (hyporesponsiveness) towards them. The system also acts by protecting against pathogens by elaborating and releasing protective peptides, cytokines, chemokines, and phagocytic cells. The IM is constantly sampling luminal contents and making molecular adjustments at its frontier.

Such a variation is not only to be found between subjects, but also the same subject can experience large variations in the microbiota over time. This can be demonstrated with vaginal microbiota as an example. The vagina, which is sterile at birth, is colonised by maternal lactobacilli within a few days. This concurs with the theory that normal human microbiota derive mainly from the mother. In most cases, this microbiota will remain prevalent throughout life; in the vagina, its presence will be mainly influenced by hormone levels. The presence of lactobacilli is strongly influenced by the availability of glycogen, the levels of which are regulated by the oestrogens. A few weeks after birth there is a reduction in the *Lactobacillus* population, to the advantage of coagulase-negative staphylococci, streptococci, *Escherichia coli* and other intestinal bacteria. Later, at puberty, the ideal conditions for multiplication of lactobacilli are restored (increased oestrogen levels and thickening of the vaginal mucosa); lactobacilli thus become the dominant vaginal microbiota in adult women. During this period the microbial population in a vaginal secretion amounts to approx. 10⁷-10⁸ CFU/g. This microbial population remains substantially unchanged until the menopause, when it is replaced by a more diverse microbiota, not unlike that of the prepuberal period, but with a considerable presence of mycoplasma and, to a lesser extent, anaerobic bacteria (e.g. *Gardnerella vaginalis*).
Various studies have attempted to characterise the vaginal lactobacilli at species level. These studies demonstrate that there is a geographical difference in the composition of the vaginal microbiota . However, bacteria belonging to what is often called the *Lactobacillus acidophilus* complex seem to constitute the dominant species at childbearing age.

Bacterial vaginosis is an aberrant state of the vaginal microbiota, which is characterized by an increase of the vaginal pH, a reduction of *Lactobacillus spp*., and an increased diversity of vaginal anaerobic bacteria and which thus may be characterized as an example of dysbiosis.. Bacterial vaginosis is associated with adverse pregnancy outcomes, upper genital tract infections and increased risk of sexual transmitted diseases.

It now has been found that a method to treat dysbiosis in a subject is advantageously performed by taking a sample of micro-organisms from the location where the dysbiosis is present, culturing these micro-organisms under conditions that favour the growth of beneficial bacterial species and/or disfavour the growth of less beneficial or pathogenic species, and, when the culture has been sufficiently cultured to have regained a more healthy balance in the presence of the micro-organisms, re-introduce a sample of said culture into the same subject. Of course it will depend on the location of the body where the sample has been taken which microbiota would be considered to be beneficial to the health and which microbiota would be considered not to be healthy. When taking the vagina as example, the most abundant bacterial genus is that of *Lactobacillus* (dominated by *L. crispatus*, *L. gasseri, L. iners, L. jensenii* and/or *L. vaginalis*), which are responsible for the lactic acid production, which is characteristic for the vaginal environment. Other lactic acid-producing bacteria that contribute to vaginal acid production by fermentation, *i.e.* species from the *genera Atopobium, Leptotrichia, Leuconostoc, Megasphaera, Pediococcus, Streptococcus* and *Weissella* may also be present. However, also several anaerobic bacterial species are frequently found in the vagina, such as the Grampositive cocci: *Atopobium vaginae, Peptostreptococcus* spp., *Staphylococcus* spp., *Streptococcus* spp., and *Bacteroides* spp., *Fusobacterium* spp., *Gardnerella vaginalis, Mobiluncus, Prevotella* spp., and Gram-negative enteric organisms, such as *Escherichia coli.*

The indigenous microbiota of the vagina differs greatly from the indigenous microbiota that can be encountered on the skin. There *Staphylococcus epidermidis, Corynebacteriae, Staphylococcus aureus, Staphylococcus warneri, Propionibacterium acnes, Pseudomonas aeruginosa, Micrococcus spp, Peptostreptococcus spp..* and *Mycobacteria* are abundant, but their presence may vary according to the exact location on the skin (e.g. the microbiota at the back of the hand may be very different from the flora found under the sole of the foot).

One location which recently has been mentioned to benefit from the administration of probiotics is the lungs, or more generally the respiratory tract. It has only recently been established (Charlson, E.S. et al., Am. J. Respir. Crit. Care Med. 184:957-963, 2011) that endogenous microbiota not only are present in the upper respiratory tracts, but also colonize the alveoli and bronchioles. As such, they form a stable environment, which can be disturbed by pathogenic, infection causing bacteria..

The community of commensal bacteria that colonize parts of the organ of the host, colonize the mucus layer and epithelial cells, which form a so-called shedding surface (for example, the mammalian intestine is covered by a single layer of epithelial cells that is renewed every 4-5 days). In other words, there is a constant turn-over of cells that are colonized by microbes in our body. Accordingly, microorganisms that adhere to the lumen will be constantly released in the lumen, and can be recovered from sputum, saliva, faeces, etc.. Samples of the indigenous microflora can also be obtained by swabbing the epithelial surface (e.g. vaginal swabs).

With respect to the present treatment of bacterial vaginosis; this is currently mostly effected by administration of antibiotics, such as metronidazole or clindamycin. However, these antibiotics are often ineffective, harmful to beneficial bacteria (such as *Lactobacillus*) and associated with a wide range of side effects and infection recurrences. Accordingly, treatment of the dysbiosis cause by bacterial vaginosis with the method according to the present invention is a major improvement in the therapy of bacterial vaginosis.

In a related embodiment cultivation or transfer of the vaginal microbiota of mothers for inoculation of babies with a Caesarean section appears advantageous. It is known that babies that have been delivered through a Caesarean section are prone to auto-immune diseases at later age, such as type 1 diabetes, Crohn's disease, multiple sclerosis, asthma, allergic rhinitis, atopic dermatitis and psoriasis (Neu, J. and Rushing, J., 2011, Clin. Perinatol. 38(2):321-331). One of the reasons for developing these auto-immune diseases is explained in the 'hygiene hypothesis' which suggests that an overly clean environment, especially in early childhood, may contribute to the development of several childhood diseases. Most current literature suggests that the gastrointestinal tract of a normal fetus is sterile. During birth and rapidly thereafter, bacteria from the mother and the surrounding environment colonize the infant's gut. It was found that infants born by Caesarean delivery had particularly low bacterial richness and diversity , even if they were breastfed. Bacteroides species were underrepresented in such infants (Azad, M.B. et al., 2013, Canad. Med. Ass. J. DOI: 10.1503/cmaj.121189). It is now an embodiment of the invention to sample the vaginal microbiota of the mother at the moment of birth, optionally culturing this sample, optionally enriching the sample in this culture, and orally administering (a part) of said (optionally cultured and optionally enriched) sample to the infant.

When a sample has been taken from a location in or on the body where there is a dysbiosis, the sample will still contain beneficial bacterial species, but they will be in the minority or overwhelmed by species that are considered to be less healthy (see e.g. Fig. 2, left two measurements). According to the invention such a sample then is cultured under conditions that favour the growth of beneficial microbiota. It will be understood that the determination of the conditions that support growth of beneficial microbiota will depend on the type of bacteria that are considered to be beneficial for the specific location of the body. As can be observed from the above, for vaginal samples, the presence of lactic acid producing bacteria, especially Lactobacillus, will be considered to be healthy, and these thus would qualify as beneficial bacteria. This then means that for culturing vaginal samples culture conditions that favour lactic acid producing bacteria would be suitable for use in the invention.

It is submitted that the skilled person would be able to choose a culture medium that would be suitable for culturing beneficial bacteria in a sample taken from any location of the human or animal body. One such medium would be a medium that favours the growth of *Lactobacillus* species, which are generally considered to be 'healthy bacteria'.

Lactobacillus-MRS Agar [introduced by De Man, J. et al., J. Appl. Bact. 23:130-135, 1960)] (LMRS AGAR) is an enriched selective medium intended for the isolation and cultivation of *Lactobacillus* found in clinical specimens and dairy and food products. LMRS AGAR is an enriched selective medium that is useful in the cultivating of *Lactobacillus* from clinical, dairy and food samples. The basis of this medium consists of peptones yeast extract and glucose. This medium is supplemented with sorbitan monooleate complex (a source for fatty acids) and magnesium for additional growth requirements. Sodium acetate and ammonium citrate are added to inhibit normal flora, such as gram-negative bacteria, oral flora and fungi. With the addition of both of these inhibiting agents, the medium has been shown to selectively improve the growth of *Lactobacillus.* The pH is adjusted to 6.3 - 6.7 to favour the growth of *Lactobacillus.* This medium is prepared, dispensed, stored and packaged under oxygen-free conditions to prevent the formation of oxidized products prior to use.

Alternatively, for the enrichment of Lactobacilli of the vaginal flora we used a so-called CDM medium as described in Geshnizgani A.M.,and Onderdonk A.B., .J Clin Microbiol. 1992 May;30(5):1323-6. In this study, a chemically defined medium that simulates female genital tract secretions was developed for the growth of the vaginal microflora. Qualitative and quantitative studies of the growth of predominant components of the vaginal microflora indicated that all vaginal isolates tested were able to grow in this defined medium. The CDM medium was adapted by replacement of hemin (source of Fe-ions) by an equimolar amount of FeSO₄.7 H₂O in order to decrease the turbidity of the medium.

As is shown in the experimental part, we have established an enrichment for *Lactobacilli* as a result of acidification of the medium from lactate production during incubation with the vaginal flora for 24 hours (*Lactobacilli* are extremely acid tolerant).

Next to *Lactobacillus* species, and depending on the location of the body from where the sample was taken, other bacteria may considered to be beneficial and selective culture media may be developed for culturing such bacteria. One example of such other micro-organisms is *Akkermansia* (e.g. A. *muciniphila*) which has recently been established to be dominantly and abundantly present in the gastro-intestinal tract and which is beneficial in the prevention of obesitas (Derrien, M. et al., Int. J. Syst. Evolut. Microbiol. 54:1469-1476, 2004; Everard, A. et al., Proc. Natl. Acad. Sci. USA early edition, 13 May 2013, 1219451110). A medium that has been specifically developed for growing *A*. *muciniphila* has been described by Derrien et al., 2004.

Depending on the origin of the microbiota sample, the culture may contain some or even many transient microbiota. These transient micro-organisms are not part of the "indigenous microbiota" and should preferably be excluded. In some cases it is complicated to discriminate between the transient and resident microorganisms, for those body sites that contain a large number of transient micro-organisms (for example in the gastro-intestinal tract). One of the criteria which may help in distinguishing (and separating) these transient bacteria from the indigenous bacteria is their incapability to adhere to human tissue.

Accordingly, exclusion of transient bacteria can be accomplished by the following independent methods, or a combination thereof. These methods may be used in addition to or in the alternative of the above described method of culturing the microbiota sample.
(i) Use of a culture medium that closely resembles the conditions of that location of the body from which the sample was derived. It is submitted that conditions that resemble the normal environment for the indigenous microbiota will favour their proliferation in comparison with micro-organisms that are only transiently present. Some evidence that specific culture conditions that resemble the local body conditions will benefit the indigenous microbiota has recently been established in a dynamic gut model, where it was demonstrated that incorporating a mucosal environment resulted in an enrichment of a sample for Lactobacilli (Van den Abbeele P, et al., Microb Biotechnol. 2012 Jan;5(1):106-15. doi: 10.1111/j.1751-7915.2011.00308.x. Epub 2011 Oct 12).
(ii) Separating the indigenous microbiota from the transient micro-organisms by assaying for their ability to bind to mucosa and/or epithelium. Such a method has been described in EP 199535, which deals with the culturing of *Lactobacillus acidophilus*. Assaying for the detection of those kind of bacteria has been described in Fakhry, S. et al., Res Microbiol. 2009 Dec;160(10):817-23. doi: 10.1016/j.resmic.2009.09.009. Epub 2009 Sep 24).
(iii) Similar to the screening for the ability to bind to mucosa or epithelium as discussed above under item ii), a screen for the presence by hybridization or PCR-based experiments for genes that encode adhesins (or possibly by the use of available antibodies), like mucus binding proteins, can be used. Many of the genes and proteins are known and are part of larger families of genes and proteins that are characterized by the possession of consensus sequences, especially for the functional parts of the protein, i.e. those parts that bind to the mucous tissue or epithelium. For instance, one of the most abundant consensus sequences is the so-called LPXTG domain which is characteristic for a cell anchor.

After sufficient culturing (part of) the culture may be prepared for re-introduction into the patient, but the culture or the remainder of it may be stored. Preferred storing conditions are storing at very low temperatures (-80°C). In such a case cryoprotectants should be added to the medium (e.g. 15% glycerol) as is common practice in the art. Storage of the culture enables the maintenance of a bacterial stock culture from which samples can constantly be thawed and used as inoculum for a new culture. This enables a long lasting supply of the culture, which enables multiple re-introductions.

If the sample has been sufficiently cultured, i.e. when there is a sufficient amount of beneficial bacteria available in the culture with respect to the amount of less beneficial bacteria, the sample is re-introduced into the body at the location from which it was derived originally. This re-introduction can be in any convenient manner and with any convenient formulation of the cultured sample.

The cultured sample may be re-introduced by formulating it as a probiotic composition. The probiotic composition may take any form as defined herein. It may be administered orally, when appropriate (e.g. in the case of intestinal microbiota), but preferably topically. When applied topically, it may be in a pure form, but it may also be applied in the form of a cream, a foam, a lotion or an ointment. Topical application may include application by inhalation, which can be advantageous e.g. when re-introducing the cultured sample into the oral cavity or for application to the respiratory tract, including the lungs. For re-introduction into the oral cavity also other oral administration forms may be applied that disintegrate in the oral cavity, e.g. through the activity of amylase in the saliva.

As will be understood by the skilled person the probiotic composition to be re-introduced may contain excipients that would be necessary or advantageous for the administration of the bacterial culture. Many excipients have already been mentioned in the definition of a probiotic composition according to this invention, but this list may be extended by any excipient, carrier or additive which is used in pharmaceutical or neutraceutical compositions. Very advantageously, dairy products, which are often used as vehicle for the classic probiotics, may be used for delivery of a probiotic composition according to the invention to the oral cavity or the gastro-intestinal tract.

Herein described is the treatment of recurring intestinal diseases, in particular *Clostridium difficile* infections. Also in this case treatment with antibiotics, including vancomycin and metronidazole, is associated with high recurrence frequencies. This likely occurs as a consequence of the diminished protective effect of the colonic microbiota and the persistence of *C*. *difficile* spores causing reinfection. As an alternative strategy, fecal microbiota transplantation (FMT) has been applied successfully to restore the healthy gut microbiota in the patient's infected colon. As already indicated in the background section, this therapeutic strategy could profit from the ability of beneficial bacteria to colonize the host, in case the donor of the microbiota would be a close relative of the patient. Cultivation and storage of fecal microbiota from the patient sampled prior to the recurrence of the infection could offer advantages over current practices, as they overcome costs and complicated logistics associated with stool transplantations. Furthermore, the sampling only takes place at the moment of the infection, which means that it takes place at a moment at which treatment is needed. The fact that the cultivated (and enriched) samples may be stored and used as a new inoculum at the moment of recurrence of the infection is a bonus that should not be neglected. Often the initial anti-infection therapy is only partly effective and the current method of the invention would be a welcome additional therapy to help overcoming the infection. However, even with such an additive therapy there will be a risk of recurrence and then a stored sample of the enriched microbiota will be advantageous.

It should be noted that the method of the present invention is truly *personalized*, but the methodology used is *generic* for each medical application. The principle is based on the selective enrichment of the individual's own beneficial bacteria from a collection of bacteria from a specific body site. Subsequent administration of the enriched culture is assumed to be much more effective in restoring the healthy microbiota compared to today's available probiotic supplements.

It is, however, also possible to have an increased generalization. In such a case the (enriched) samples of multiple subjects are collected and cultured further together in order to propagate a relatively representative microbiota for all subjects. Such a 'generalized' propagated microbiota population will be less personalized than the culture obtained by the above described method, but it will be sufficiently effective for a larger number of people. Especially in the case where the dysbiosis is acute and developing aggressively, i.e. when there is insufficient time to obtain and culture a sample as described above, the possibility of having such a 'generalized' sample that can be administered without delay can be of enormous value, especially to at least minimize the symptoms that are caused by the dysbiosis.

Although the above has been written with a special emphasis to the human situation, it is submitted that the present invention can also be advantageously used in veterinary applications. Especially of interest are application to intestinal health, oral health and vaginal health in all kind of economically important species, such as cattle, horses, poultry, pigs, dogs, cats, etc. Two specific examples of such applications are the application for intestinal health in broilers (including lay hens and also embryo's in eggs) and intestinal health in pigs and piglets. In both cases lactobacilli have been found to dominate the intestinal microbiota and are suspected to be positively related to health. Further, only a few lactobacilli species are involved, which means that these examples are very similar to the human vaginal situation as described above (and in the examples).

The invention is further illustrated by the following examples. The examples are not to be interpreted as limiting the scope of the invention in any way.

### EXAMPLES

### Example 1: Enrichment of vaginal microbiota

### Sampling

In women with a high risk sexual behavior, routine screening for sexually transmitted diseases (STD) at the clinic includes a standard cervical examination. During routine sampling a cotton swab was used to obtain mucus and resuspended in screw-cap coded tubes with Amies transport medium (to which additional 15% glycerol and cysteine solution has been added). Immediately thereafter, the tubes were placed in liquid nitrogen and transferred for storage at - 80°C. Gram stain, Amsel criteria, and STD status are obtained at the clinic as usual for routine purposes. A prevalence of bacterial vaginosis (BV) is usually observed in 50% in the sampled population of women with a high risk sexual bahavior.

Nugent scoring was carried out on all samples prior to storage. Nugent scoring is a Gram stain scoring system, first described in 1991 by R.P Nugent (J. Clin. Microbiol. 29:297-301, 1991) for pap tests to diagnose BV.. The Nugent score is calculated by assessing for the presence of large Gram-positive rods (*Lactobacillus morphotypes*; decrease in *Lactobacillus* scored as 0 to 4), small Gram-variable rods (*Gardnerella vaginalis* morphotypes; scored as 0 to 4), and curved Gram-variable rods (*Mobiluncus spp.* morphotypes; scored as 0 to 2) and can range from 0 to 10. A score of 7 to 10 is consistent with BV.

In this study we selected samples with a Nugent scoring ranging from 8 -10; and as a control for a "healthy state" samples of a Nugent scoring from 0-3 were included.

### Enrichment

Screw -cap tubes containing the resuspended vaginal swabs were taken from the -80°C freezer and thawed at room temperature under anaerobic conditions. A volume 130 ul of the suspension was transferred to 13 ml of modified CDM medium (Geshnizgani, A. et al., J. Clin. Microbiol. 30(5):1323-1326, 1992) in a 50 ml Corning tube (modification by replacement of hemin (source of Fe-ions) by an equimolar amount of FeSO₄. 7 H₂O in order to decrease the turbidity medium)., followed by incubation for 24 hours at 37°C at 100 rpm under anaerobic conditions.

### Species determination of (enriched) microbiota by bar-coded 16S - amplicon sequencing

Quantitative 16S-PCR was performed to determine the relative amount of bacterial template in the isolated DNA samples (Biesbroek, G. et al. PLoS One, 7:e32942, 2012) prior to the generation of a 16S rRNA gene amplicon library spanning variable regions V5-V7 (Bogaert, D. et al., PLoS One 6: :e17035, 2011). Sequence analysis of the amplicon library was performed on a 454 GS-FLX-Titanium Sequencer (Life Sciences (Roche), Branford, CT). FASTA-formatted sequences and corresponding quality scores were extracted from the .sff data file generated by the GS-FLX Titatium sequencer using the GS Amplicon software package (Roche, Branford, CT) and processed using modules implemented in the Mothur v. 1.22.2 software platform (Schloss, P. et al., Appl Environ Microbiol 75: 7537-41, 2009) . Sequences were de-noised using a pseudo-single linkage algorithm with the goal of removing sequences that are likely due to pyrosequencing errors ("pre.cluster" command) (Huse, S. et al., Environ. Microbiol. 12:1889-1898, 2010). Potentially chimeric sequences were detected and removed using the "chimera.uchime" command (Edgar, R. et al., Bioinformatics 27:2194-2200, 2011). High quality aligned sequences were classified using the RDP-II naive Bayesian Classifier (Wang, O. et al., Appl Environ Microbiol 73:5261-5267, 2007) . Aligned sequences were clustered into OTUs (defined by 100% similarity) using the average linkage clustering method.

### Results

The 454 sequence analysis confirmed the presence of BV-associated genera in the samples with the Nugent scoring from 8-10 (including *Gardnerella, Bacteroides, Parvimonas, Atopobium, Sneathia, Prevotella, Dialister,* and *Megasphaera*) and the presence of species previously associated with a "healthy state" for Nugent scoring 0-3 (including *Lactobacillus crispatus, Lactobacillus gasseri* and *Lactobacillus jensenii*). This confirmed findings as previously described (Ravel et al, PNAS Early Edition, 108:4680-4687 2010.

The enrichment procedure of a BV microbiota led to a suppresion of BV-associated microflora and the accumulation of Lactobacilli. For example, in one case we obtained in an increase of total lactobacilli from 18% to 96% (see below summary of table of most abundant species; the full table including 2 replicates from t = 0 and t = 24

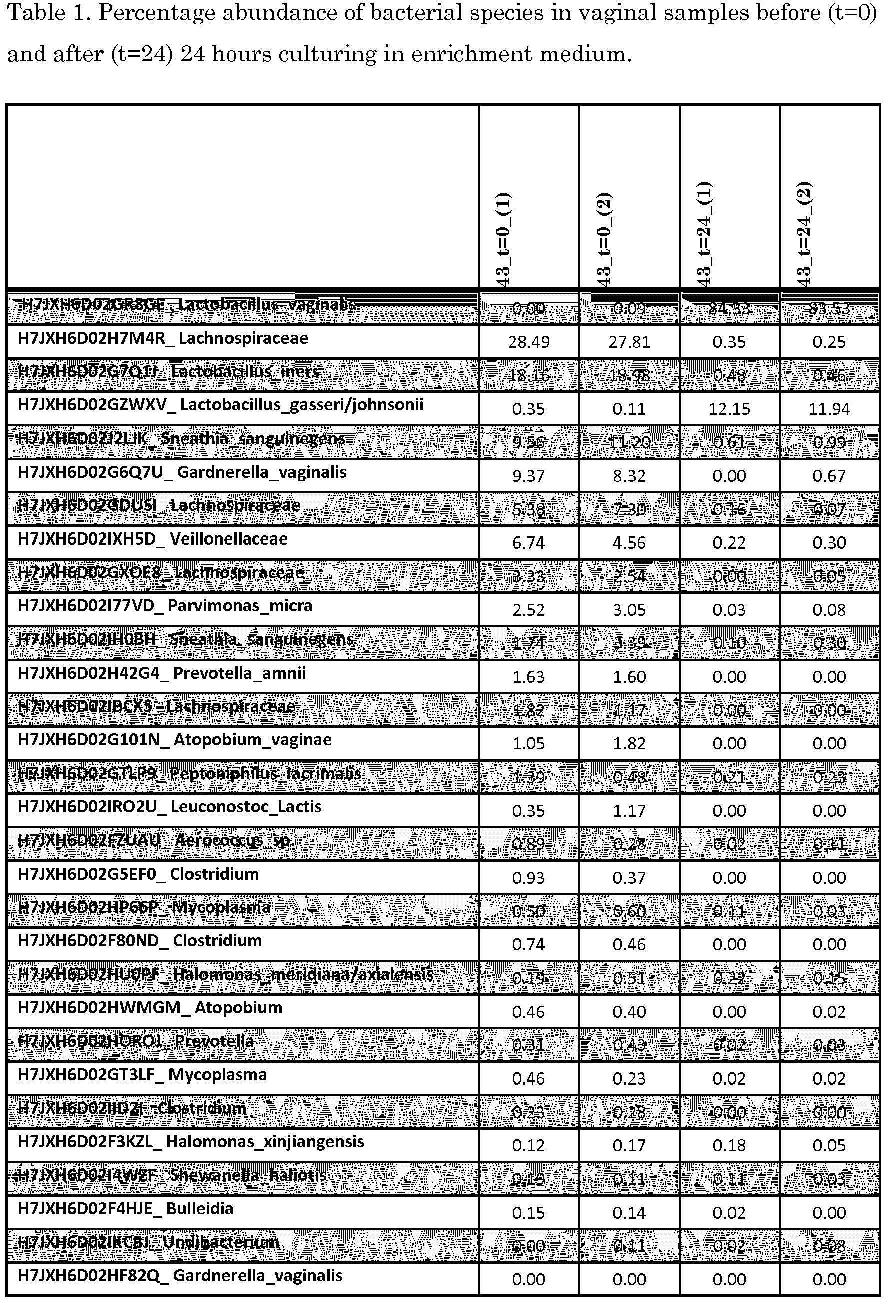

Herein describes is
1. Method for treatment of a dysbiotic condition in a subject comprising the steps of:
   a. obtaining a sample from said subject wherein said sample is obtained from the location of the dysbiotic condition and wherein said sample comprises micro-organisms;
   b. *in vitro* culturing of said sample under conditions that favour beneficial micro-organisms;
   c. re-introducing a sample of said cultured micro-organisms into said subject.
2. Probiotic composition for use in the treatment of a dysbiotic condition in a subject, wherein said composition has been obtained by culturing a sample comprising micro-organisms from the location of the dysbiotic condition from said subject under conditions that favour beneficial micro-organisms.
3. Probiotic composition according to point 2, wherein said probiotic composition comprises additional excipients.
4. Probiotic composition according to point 3, wherein said composition is suitable for administration to the location of the dysbiotic condition.
5. Method according to point 1 or probiotic composition according to claim 2, wherein said subject is a human.
6. Method according to point 1 or probiotic composition according to claim 2, wherein said subject is an animal, preferably chosen from the group of cattle, horses, poultry, pigs, dogs and cats.
7. Method according to point 1 or probiotic composition according to claim 2, wherein said location of the probiotic condition is chosen from the intestine, the vagina, the oral cavity, the respiratory tract and the skin.
8. Method according to point 1 or probiotic composition according to claim 2, wherein the micro-organisms in said sample are bacteria.
9. Method according to point 1 or probiotic composition according to claim 2, wherein the beneficial micro-organisms are selected from the group of *Lactobacilli, Bifidobacilli* and *Akkermansia..*
10. Method according to point 1 or probiotic composition according to claim 2, wherein the in vitro culture is maintained for more than two days.
11. Method according to point 1 or probiotic composition according to claim 2, wherein the re-introduction is performed by oral or topical administration.
12. Method according to point 1, wherein said cultured, enriched sample is stored and in which a sample of said stored sampled is used for administration to said subject in case of a recurrent dysbiotic condition, such as a recurrent infection.
13. Method to prevent the occurrence of an auto-immune disease in children delivered through Caesarean section, comprising:
   a. Taking a vaginal microbiota sample from the mother at the time of delivery, or shortly before or after this time;
   b. Optionally culturing said sample, during which culture said sample may optionally be enriched for beneficial micro-organisms;
   c. Administration of at least a part of said sample obtained from step a or step b to the new-born child, preferably by oral administration.

## Claims

1. Probiotic composition for use in the treatment of bacterial vaginosis in a subject, wherein said composition has been obtained by *in vitro* culturing a sample comprising bacteria from the vagina from said subject, wherein said subject already suffers from bacterial vaginosis, under conditions that favour beneficial bacteria, wherein said beneficial bacteria are *Lactobacilli.*

2. Probiotic composition for use according to claim 1, wherein said probiotic composition comprises additional excipients.

3. Probiotic composition for use according to claim 1 or 2, wherein said composition is suitable for administration to the vagina.

4. Probiotic composition for use according to any of the previous claims, wherein said subject is a human.

5. Probiotic composition for use according to any of the previous claims, wherein said culturing is performed for more than two days.

6. Probiotic composition for use according to any of the previous claims, wherein said cultured sample is stored for administration to said subject in case of a recurrent bacterial vaginosis.

## Patentansprüche

1. Probiotische Zusammensetzung zur Verwendung bei der Behandlung von bakterieller Vaginose bei einem Subjekt, wobei die Zusammensetzung durch *in vitro* Kultivieren einer Probe, umfassend Bakterien aus der Vagina von dem Subjekt, erhalten worden ist, wobei das Subjekt bereits an bakterieller Vaginose leidet, unter Bedingungen, die vorteilhafte Bakterien begünstigen, wobei die vorteilhaften Bakterien *Lactobacilli* sind.

2. Probiotische Zusammensetzung zur Verwendung nach Anspruch 1, wobei die probiotische Zusammensetzung zusätzliche Hilfsstoffe umfasst.

3. Probiotische Zusammensetzung zur Verwendung nach Anspruch 1 oder 2, wobei die Zusammensetzung zur Verabreichung in die Vagina geeignet ist.

4. Probiotische Zusammensetzung zur Verwendung nach einem der vorhergehenden Ansprüche, wobei das Subjekt ein Mensch ist.

5. Probiotische Zusammensetzung zur Verwendung nach einem der vorhergehenden Ansprüche, wobei das Kultivieren für mehr als zwei Tage durchgeführt wird.

6. Probiotische Zusammensetzung zur Verwendung nach einem der vorhergehenden Ansprüche, wobei die kultivierte Probe zur Verabreichung an das Subjekt im Falle einer wiederkehrenden bakteriellen Vaginose aufbewahrt wird.

## Revendications

1. Composition probiotique pour une utilisation dans le traitement d'une vaginose bactérienne chez un sujet, dans laquelle ladite composition a été obtenue par culture *in vitro* d'un échantillon comprenant des bactéries provenant du vagin dudit sujet, dans laquelle ledit sujet souffre déjà d'une vaginose bactérienne, dans des conditions qui favorisent des bactéries bénéfiques, dans laquelle lesdites bactéries bénéfiques sont *Lactobacilli.*

2. Composition probiotique pour une utilisation selon la revendication 1, dans laquelle ladite composition probiotique comprend des excipients supplémentaires.

3. Composition probiotique pour une utilisation selon la revendication 1 ou 2, dans laquelle ladite composition est appropriée pour une administration dans le vagin.

4. Composition probiotique pour une utilisation selon l'une quelconque des revendications précédentes, dans laquelle ledit sujet est un humain.

5. Composition probiotique pour une utilisation selon l'une quelconque des revendications précédentes, dans laquelle ladite culture est réalisée pendant plus de deux jours.

6. Composition probiotique pour une utilisation selon l'une quelconque des revendications précédentes, dans laquelle ledit échantillon cultivé est stocké pour une administration audit sujet dans le cas d'une vaginose bactérienne récurrente.
